Europäisches Patentamt

European Patent Office

Office européen des brevets

(11) Veröffentlichungsnummer: **0 093 405**
**A2**

# EUROPÄISCHE PATENTANMELDUNG

(21) Anmeldenummer: 83104147.0

(22) Anmeldetag: 28.04.83

(51) Int. Cl.³: **A 61 B 19/00**
**A 61 C 17/02**

(30) Priorität: 29.04.82 DE 3216017

(43) Veröffentlichungstag der Anmeldung:
09.11.83 Patentblatt 83/45

(84) Benannte Vertragsstaaten:
AT BE CH DE FR GB IT LI LU NL SE

(71) Anmelder: Lehner, Bernard, Dr.med.dent.
Am Haseneck 1
D-6780 Pirmasens(DE)

(72) Erfinder: Lehner, Bernard, Dr.med.dent.
Am Haseneck 1
D-6780 Pirmasens(DE)

(74) Vertreter: Sandmann, Joachim, Dr.
Hirtenstrasse 19
D-8012 Ottobrunn(DE)

(54) Vorrichtung zum Beträufeln eines Osteotomiegebietes mit Kühlflüssigkeit.

(57) Eine Kanüle 1 zum Beträufeln eines Osteotomiegebietes mit einer sterilen Kühlflüssigkeit weist ein inneres Ende 5 zur lösbaren Verbindung mit einem Schlauch 2, der zu einem Kühlflüssigkeitsbehälter führt, und ein Gewinde 7 auf, mit dem die Kanüle in eine Gewindebohrung 12 eines Handstücks 3 einschraubbar ist. Das Handstück 3 nimmt das der Kanüle 1 benachbarte Schlauchstück 6 auf und ist mit einer am Außenumfang des Schlauchstücks 6 angreifenden Quetschscheibe 15 versehen, die in Längsrichtung des Handstücks 3 verstellbar ist und dadurch über ein mehr oder minder starkes Zusammenquetschen des Schlauchstücks 6 den Kühlflüssigkeitsdurchsatz steuert.

FIG. 2

Croydon Printing Company Ltd.

Dr. Bernhard Lehner

- ; -

**B e s c h r e i b u n g :**

Die Erfindung betrifft eine Vorrichtung zum Beträufeln eines
Osteotomiegebietes mit einer sterilen Kühlflüssigkeit, bestehend aus einer Kanüle, die über einen Schlauch, der lösbar mit dem inneren Kanülenende verbindbar ist, an einen
die Kühlflüssigkeit enthaltenden Behälter anschließbar ist,
und mit einer Dosiereinrichtung zur Änderung des Kühlflüssigkeitsdurchsatzes versehen ist.

Eine derartige Vorrichtung, die insbesondere im Bereich der
Kieferchirurgie zur Anwendung kommt, ist bereits bekannt
(US-PS 3 949 753). Dort wird die Kanüle aus Metall am Halter
des Werkzeugs für die Knochenbearbeitung festgeklemmt. Der
Schlauch ist auf das hintere Ende der Kanüle aufgesteckt und
in seinem an die Kanüle anschließenden Abschnitt mit einem
Drosselventil versehen, das die Dosiereinrichtung zur Änderung des Kühlflüssigkeitsdurchsatzes bildet. Das andere
Schlauchende erstreckt sich in einen Abgabebehälter und ist
an eine zusammendrückbare Kühlflüssigkeitsflasche im Abgabebehälter angeschlossen. In den Abgabebehälter mündet eine
von einer Druckquelle ausgehende Druckleitung, über die der
Abgabebehälter unter Druck gesetzt wird, um die Kühlflüssigkeit durch den Schlauch zur Kanüle hin auszudrücken.

Diese bekannte Vorrichtung ist aus mehreren Teilen zusammengesetzt und dementsprechend aufwendig. Ihr Einsatz ist
daher in vielen Fällen aus wirtschaftlichen Erwägungen nicht
gerechtfertigt, so beispielsweise in einer üblichen Zahnarztpraxis, in der kieferchirurgische Behandlungen nur gelegentlich anfallen. Hier wird allgemein mit geringerem
instrumentellem und personellem Aufwand gearbeitet, und
dementsprechend besteht ein Bedarf nach einer einfachen
bedienungsfreundlichen Vorrichtung, die trotzdem ihre Funktion

./.

- 3 -

voll erfüllt und zuverlässig arbeitet.

Bei kieferchirurgischen Eingriffen in einer Zahnarztpraxis
wird bisher zur Kühlung des Operationsgebietes eine Wasserspritze verwendet, die durch die Operationsassistenz aus
einem Standzylinder mit steriler Kühlflüssigkeit gefüllt
wird. Die hierbei verwendeten Instrumente bedeuten zwar
keinen ins Gewicht fallenden Aufwand, zumal eine Zahnarztpraxis ohnehin mit einer entsprechenden Ausrüstung versehen
ist, die Arbeitsweise ist jedoch unergonomisch und führt zu
für den Patienten wie für den Operateur unliebsamen Erschütterungen wie auch Zeitverlusten während der Operation. Allerdings besteht gegenüber der vorbeschriebenen bekannten Vorrichtung insoweit ein Vorteil, als die verwendete Spritze vom
Werkzeug für die Knochenbearbeitung getrennt ist und daher
das Gesichtsfeld des Operateurs nicht einschränkt, was bei
der bekannten Vorrichtung insbesondere bei Operationen im
distalen Bereich der Mundhöhle zu befürchten ist. Zwar kann
die am Werkzeughalter angeklemmte Kanüle ggf. abgenommen
werden, sie ist dann jedoch mangels einer Halterung bzw.
eines Handstücks unhandlich und von der Operationsassistenz
nur schwierig anzuwenden und zu bedienen. Im übrigen ist
auch eine Änderung der Kühlungsintensität bzw. des Kühlflüssigkeitsdurchsatzes während der Operation nicht mit der
gewünschten Leichtigkeit im Wege der Einhandbedienung vorzunehmen, da die den Werkzeughalter haltende Hand nicht gleichzeitig auch das Drosselventil am Schlauch betätigen kann.
Schließlich fehlt es wegen des in das Schlauchinnere hineinführenden Drosselventils auch an der wünschenswerten einfachen
Sterilisierbarkeit der Vorrichtung.

Der Erfindung liegt die Aufgabe zugrunde, die eingangs genannte Vorrichtung einfach und preiswert sowie bedienungsfreundlich auszubilden, um sie auch für eine normale Zahnarztpraxis geeignet zu machen.

./.

Diese Aufgabe wird erfindungsgemäß dadurch gelöst, daß die Kanüle eine Befestigungseinrichtung aufweist, durch die sie lösbar mit einem eine zugehörige Verbindungseinrichtung aufweisenden Handstück verbunden ist, die das der Kanüle benachbarte Schlauchstück aufnimmt und mit der Dosiereinrichtung versehen ist, die als am Außenumfang des Schlauchstücks angreifende Quetscheinrichtung ausgebildet ist.

Bei dieser Ausbildung bildet die mit dem Handstück versehene Kanüle ein vom Werkzeug für die Knochenbearbeitung unabhängiges Hilfswerkzeug, so daß das Knochenbearbeitungswerkzeug ohne kühlungsspezifische Sichtbehinderungen zum Einsatz kommen kann. Das der Kühlung dienende Hilfswerkzeug läßt sich auf Grund des vorgesehenen Handstücks einfach und auch über einen längeren Zeitraum verkrampfungsfrei mit einer Hand einsetzen bzw. handhaben, wobei zugleich mit dieser Hand die Dosiereinrichtung betätigt werden kann, um den Kühlflüssigkeitsdurchsatz den jeweiligen Erfordernissen anzupassen und somit eine optimale Kühlung sowie ggf. Spülung zu erzielen. Dabei kann ein sich ändernder Zulaufdruck der Kühlflüssigkeit ohne weiteres ausgeglichen werden, so daß unter wesentlicher Vereinfachung der Gesamtvorrichtung auf einen besonderen Abgabebehälter, in den ein Druckmittel zum Ausdrücken der sterilen Kühlflüssigkeit aus ihrer flexiblen Verpackung eingeleitet wird, verzichtet werden kann. Vielmehr kann das hintere Ende des Schlauches direkt an die oberhalb der Operationsstelle angeordnete Kühlflüssigkeitsflasche angeschlossen werden, die das benötigte Kühlflüssigkeitvolumen, beispielsweise 1 Liter, enthält, um die Operation ohne Unterbrechung durchführen zu können. Ferner läßt sich die Vorrichtung leicht zusammenbauen und zerlegen, um ggf. die Kanüle und den Schlauch zwecks Wiederverwendung zu sterilisieren. Diese Teile können jedoch auch als Wegwerfteile für die einmalige Verwendung vorgesehen sein, ohne daß dieses zu einer ins Gewicht fallenden Kostenbelastung führt. Es ist daher ersichtlich, daß die Erfindung zu einer

./.

- : -

preiswerten Vorrichtung führt, die auch dort mit Vorteil eingesetzt wird, wo sie nur gelegentlich gebraucht wird.

Zweckmäßige Ausgestaltungen und Weiterbildungen ergeben sich
aus den Unteransprüchen. Die danach vorgesehene Dosiereinrichtung mit einer am Außenumfang eines Schlauchstücks angreifenden Quetschscheibe ist als solche bereits bekannt
(DE-OS 19 34 337).

Ein Ausführungsbeispiel der Erfindung wird nachfolgend anhand einer schematischen Zeichnung näher erläutert. Es zeigt:

Fig. 1 die zusammengebaute Vorrichtung mit der in zurückgezogener Ruhestellung befindlichen Quetschscheibe in
       Seitenansicht;

Fig. 2 eine teilweise vertikal geschnittene, im übrigen Fig.1
       entsprechende Darstellung mit in Betriebsstellung befindlicher Quetschscheibe und mit abgenommener Kanüle
       und

Fig. 3 die Einschaltung eines zusammendrückbaren Ballons mit
       einer Absperrvorrichtung in die Leitung zum Zuführen
       der Kühlflüssigkeit.

Die Vorrichtung besteht im wesentlichen aus einer Kanüle 1,
einem Schlauch 2 und einem griffigen Handstück 3 mit einer
Dosiereinrichtung 4.

Die Kanüle 1 weist ein inneres Ende 5 auf, das in das benachbarte, vom Handstück 3 aufgenommene Schlauchstück 6 bzw.
Ende des Schlauchs 2 abdichtend einschiebbar ist. Ferner
weist die Kanüle 1 im Anschluß an ihr inneres Ende 5 ein
Außengewinde 7 und im Anschluß daran eine Verdickung 8 mit
zwei Schraubflügeln 9 auf.

Das Handstück 3 ist in seiner der Kanüle 1 zugewandten Stirnfläche 10 mit einer ein Gewinde 11 aufweisenden Gewindebohrung 12 versehen, in welche die Kanüle 1 mit ihrem Gewinde 7 einschraubbar ist.

./.

Koaxial zur Gewindebohrung 12 erstreckt sich als deren Fortsetzung eine Schlauchbohrung 13 durch das Handstück 3. Diese nimmt wie dargestellt das Schlauchstück 6 auf.

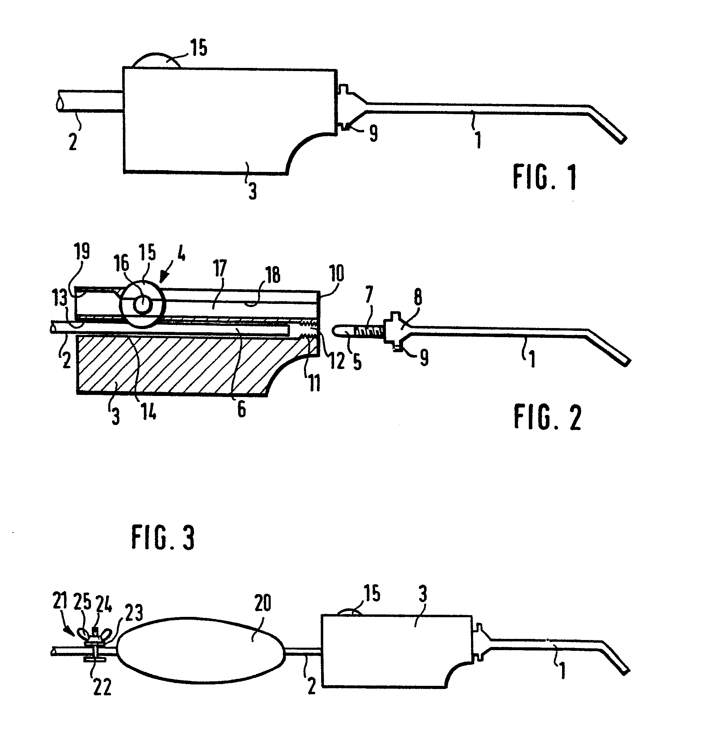

Die Dosiereinrichtung 4 umfaßt eine Andrückfläche 14 an der Unterseite des Schlauchstücks 6 sowie eine der Andrückfläche 14 gegenüberliegende Quetschscheibe 15 mit zwei seitlichen Achsstutzen 16, die jeweils in einer Führungsnut 17 des Handstücks 3 laufen. Die oberen Führungskanten 18 der beiden Führungsnuten verlaufen in Richtung auf die Kanüle 1 etwas nach unten geneigt, so daß bei einer Verlagerung der nach oben aus dem Handstück 3 vorstehenden Quetschscheibe 15 in Richtung auf die Kanüle 1 die Quetschscheibe 15 das Schlauchstück 6 zunehmend zusammendrückt, um seinen Durchtrittsquerschnitt zu verengen und dadurch den Kühlflüssigkeitsdurchsatz herabzusetzen. Hierdurch kann die Kühlflüssigkeit feinfühlig dosiert und nach Bedarf tropfenweise durch die Kanüle 1 abgegeben werden. Wird die Quetschscheibe 15 bis in eine Nuterweiterung 19 zurückbewegt, was der Stellung in Fig. 1 entspricht, so kann sich das Schlauchstück 6 unter vollständiger Öffnung seines Durchströmquerschnitts elastisch aufweiten. In dieser Ruhestellung der Quetschscheibe 15 kann der Schlauch 2 mit seinem Schlauchstück 6 in die Schlauchbohrung 13 eingeführt bzw. nach dem Lösen vom inneren Ende 5 der Kanüle 1 aus der Schlauchbohrung 13 herausgezogen werden.

Ggf. kann die Gewindebohrung 12 einen Durchmesser aufweisen, der den Durchmesser der Schlauchbohrung 13 übersteigt, so daß das Schlauchstück 6 vollständig durch das Handstück 3 hindurchgeführt werden kann, um die Verbindung zwischen dem Schlauchstück 6 und der Kanüle 1 außerhalb des Handstücks 3 vorzunehmen und danach die Kanüle 1 in das Handstück 3 einzuschrauben. In diesem Falle wird das Gewinde 7 zweckmäßigerweise an einer axialen Verlängerung der Verdickung 8 vorgesehen.

./.

Die Kanüle 1 kann als preiswertes Kunststoffteil hergestellt
sein, was sich insbesondere im Falle einmaliger Verwendung
zur Vermeidung eines Sterilisieraufwandes anbietet. Entsprechendes gilt für den Schlauch 2, der nur eine vergleichsweise geringe Länge zu haben braucht, um genügend Bewegungsspielraum für die Handhabung der Vorrichtung zu schaffen.
Auch das Handstück 3, das mit weiteren nicht dargestellten
Hohlräumen zur Einsparung an Material und Gewicht versehen
sein kann, kann ebenso wie die Quetschscheibe 15 als billiges
Formteil aus Kunststoff hergestellt sein.

Gemäß Fig. 3 ist der zum Handstück 3 führende Schlauch 2 mit
einem von Hand zusammendrückbaren Ballon 20 versehen, der ein
Fassungsvermögen von beispielsweise etwa 100 ml hat. Auf der
dem Handstück 3 mit der Kanüle 1 abgewandten Seite des Ballons 20 ist der Schlauch 2 mit einer Absperrvorrichtung 21
versehen, bei der es sich beispielsweise um eine einfache und
leicht betätigbare Kreuzklemme handeln kann. Wie dargestellt
sind zwei federnd miteinander verbundene Klemmabschnitte 22
und 23 auf gegenüberliegenden Seiten des Schlauchs 2 vorgesehen, die mittels einer Betätigungseinrichtung, beispielsweise ein vom Klemmabschnitt 22 durch den Klemmabschnitt 23
aufragender Gewindebolzen 24 mit aufgeschraubter Flügelmutter 25, zusammendrückbar sind.

Auf Grund dieser einfachen Zusatzmaßnahmen, die keinen
wesentlichen Mehraufwand bedeuten, kann gewünschtenfalls
nach dem Schließen der Absperrvorrichtung 21 durch Zusammendrücken des mit Kühlflüssigkeit gefüllten Ballons 20 in der
Hand eine Druckspülung mit erhöhtem Kühlflüssigkeitsdurchsatz vorgenommen werden, wobei das Schlauchstück 6 im Bereich der Quetschscheibe 15 natürlich geöffnet sein muß.

Vorrichtung zum Beträufeln eines
Osteotomiegebietes mit Kühlflüssigkeit

A n s p r ü c h e :

1. Vorrichtung zum Beträufeln eines Osteotomiegebietes mit
einer sterilen Kühlflüssigkeit, bestehend aus einer Kanüle,
die über einen Schlauch, der lösbar mit dem inneren Kanülenende verbindbar ist, an einen die Kühlflüssigkeit enthaltenden Behälter anschließbar ist, und mit einer Dosiereinrichtung zur Änderung des Kühlflüssigkeitsdurchsatzes
versehen ist, dadurch g e k e n n z e i c h n e t , daß
die Kanüle (1) eine Befestigungseinrichtung (7) aufweist,
durch die sie lösbar mit einem eine zugehörige Verbindungseinrichtung (11) aufweisenden Handstück (3) verbunden ist,
das das der Kanüle (1) benachbarte Schlauchstück (6) aufnimmt und mit der Dosiereinrichtung (4) versehen ist, die
als am Außenumfang des Schlauchstücks (6) angreifende
Quetscheinrichtung ausgebildet ist.

2. Vorrichtung nach Anspruch 1, dadurch gekennzeichnet, daß
die Befestigungseinrichtung an der Kanüle (1) und die Verbindungseinrichtung am Handstück (3) miteinander verschraubbare Gewinde (7 und 11) sind.

./.

3. Vorrichtung nach Anspruch 2, dadurch gekennzeichnet, daß das Gewinde (7) an der Kanüle (1) ein Außengewinde ist, dem eine Gewindebohrung (12) in der Stirnfläche (10) des Handstücks (3) zugeordnet ist.

4. Vorrichtung nach Anspruch 2 oder 3, dadurch gekennzeichnet, daß die Kanüle (1) mit Schraubflügeln (9) versehen ist.

5. Vorrichtung nach einem der Ansprüche 1 bis 4, dadurch gekennzeichnet, daß die Dosiereinrichtung (4) eine Andrückfläche (14) für das Schlauchstück (6) und eine dieser auf der anderen Seite des Schlauchstücks (6) gegenüberliegende über den Außenumfang des Handstücks (3) vorstehende Quetschscheibe (15) mit seitlichen Achsstutzen (16) aufweist, die in Führungsnuten (17) des Handstücks (3) laufen, die sich in Längs- bzw. Achsrichtung leicht geneigt zur Andrückfläche (14) erstrecken.

6. Vorrichtung nach einem der Ansprüche 1 bis 5, dadurch gekennzeichnet, daß in den zum Handstück (3) führenden Schlauch (2) ein zusammendrückbarer Ballon (20) eingefügt ist, vor dem eine Absperrvorrichtung (21) zum Absperren des Schlauchs (2) angeordnet ist.

7. Vorrichtung nach Anspruch 6, dadurch gekennzeichnet, daß der Ballon (20) eine solche Größe aufweist, daß er in einer Hand zusammendrückbar ist.

FIG. 1

FIG. 2

FIG. 3